# EUROPEAN PATENT APPLICATION

(11) **EP 3 348 302 A1**
(43) Date of publication of application: **18.07.2018**
(21) Application number: 18275005.9
(22) Date of filing: 12.01.2018
(51) Int. Cl.: A61M 25/09, A61M 25/00, A61B 1/005, A61B 1/00

(54) **MODULAR MEDICAL GUIDE WIRE ASSEMBLY**

(30) Priority: 13.01.2017 US 201715405883; 07.12.2017 GB 201720416
(71) Applicant: Cook Medical Technologies LLC, Bloomington, IN 47404 (US)
(72) Inventor: HANSEN, Palle Munk, 4632 Bjaeverskov (DK); BODEWADT, Tue Thuren, 2680 Solroed Strand (DK)
(74) Representative: Williams Powell

(57) **Abstract**

A guide wire assembly (50) for medical use includes at least a portion (90) which is formed of a plurality of inter-cooperating elements (100) forming a flexible section (90) of the guide wire assembly (50), and specifically a section that can have varying flexibility. A control device (58) passes through a lumen extending for the whole length of the guide wire (50) and is typically fixed at a distal end (54) of the assembly (50). The control device (58) is used to change the flexibility of the guidewire assembly and in some embodiments to lock it in position. The elements (100) have tapering internal lumens for accommodating the control device (58), particularly when the guide wire (50) is bent.

## Description

### Technical Field

The present invention relates to a modular medical guide wire assembly for use, for example, in endoluminal medical procedures.

### Background of the Invention

Endoluminal medical procedures are now common in many countries as they can significantly reduce clinical procedure times, can effect localised treatments, and can significantly reduce patient trauma and convalescence. Many techniques, including the well-established Seldinger procedure, make use of a guide wire which is fed from a remote percutaneous entry point through the patient's vasculature to the site to be treated. The treatment could be in a major vessel, such as the aorta or vena cava, but increasingly may be within a very small and delicate vessels such as the cerebral vessels.

Generally, the guide wire is the first element of an introducer assembly that is positioned at the treatment site, with the other elements of the assembly being fed subsequently over the guide wire from the same percutaneous entry point. For this purpose, it is important that the guide wire is sufficiently soft, particularly at its distal end, in order to be able to curve through the patient's vasculature and into and through any branch vessels. However, it is also important for the guide wire to exhibit some strength, or rigidity, so that it can effectively guide the subsequently deployed elements of the introducer assembly through the tortuous paths of the patient's vasculature. Often, this leads to the guide wire having to exhibit contradictory characteristics of softness and strength. In some circumstances, such as in neurological applications, it is not possible or optimal to compromise on softness and strength, with the result that it becomes necessary to use in the same procedure a plurality of guide wires each having different characteristics.

It is known to have modular guide wires formed of a plurality of components fitted to a control wire which can be loosened to allow the components to slide relative to one another and which can be tightened so as to lock the components together.

Examples of some known guide wire assemblies can be found in US-2012/191012, US-3625200, US-2003/105415, US-2004/0254450, US-2009/099554, US-5,902,254, US-6,682,493, US-4294260, and US-4,955,384, while an example of an endoluminal surgical instrument can be found in US-2012/0143175.

### Summary of the Invention

The present invention seeks to provide an improved medical guide wire. The term guide wire or guide wire device is used herein in its general form. A variety of types of guide wire are known, such as: of simple wire core form; as an assembly of a wire core and outer layer that may be a polymer coating or coiled wire tubing; as a series of interlinked elements able to pivot relative to one another on a wire carrier; and so on. The term guide wire is therefore to be understood to refer to the guide element of an introducer assembly which is used to guide the subsequent passage of introducer assembly components such as catheters, medical device carriers, diagnostic tools, protective sheaths and so on.

According to an aspect of the present invention, there is provided a medical guide wire of elongate form and having a longitudinal dimension, a distal end and a proximal end; at least a portion of the guide wire being formed of a plurality of inter-cooperating elements disposed longitudinally in series, the inter-cooperating elements having cooperating facing surfaces able to slide relative to one another so as to cause the guide wire to be bendable in at least one lateral dimension; each inter-cooperating element having a bore extending from one end to the other end thereof, the bore having a taper from said one end to the other end; and a control device extending through the bores of the inter-cooperating elements, the control device having at least one operating state enabling the inter-cooperating elements to slide relative to one another.

The provision of an internal bore in the inter-cooperating elements provides space for the control device when the elements slide relative to one another so as not to be in a straight configuration, that is when they are pivoted or tilted relative to one another. This increases the flexibility of the guide wire and also enables to control device to be tightened, typically into a different operating state, with no or little straightening force being imparted to the inter-cooperating elements. As a result, the guide wire is more flexible and conformable relative to prior art arrangements.

Preferably, the control device has a first operating state in which the inter-cooperating elements can slide relative to one another and the guide wire is relatively flexible, and a second operating state limiting sliding of the inter-cooperating elements and the guide wire is relatively less flexible. More preferably, the control device has an operating state preventing sliding of the inter-cooperating elements and which causes the guide wire to be stiff. In this embodiment, the control device can act as a locking device to lock the guide wire in a bent or straight configuration, typically in dependence upon the anatomy of the vessel or vessels in which the guide wire is in use positioned.

The cooperating facing surfaces may be textured, toothed or friction surfaces. That is, in these embodiments, the surfaces do not slide relative to one another when pushed together. With such a structure, a longitudinally compressive force applied to the guide wire will cause the elements to lock together, thereby making the guide wire stiff or rigid.

In the preferred embodiment, at least one of the cooperating facing surfaces is curved. Advantageously, both of the cooperating facing surfaces are curved.

Advantageously, at least one of the facing cooperating surfaces is rounded. It or they may be part-circular and optionally respectively convex and concave.

Preferably, each inter-cooperating element includes at one end a convex surface and at the other end a concave surface.

In an embodiment, the inter-cooperating elements include a first end with a rounded outer surface and a second end with an inner surface, the second end being sized to envelop the first end of an adjacent inter-cooperating element. Advantageously, the internal surface of the second end is in the form of a chamber having an opening of a width or diameter less than a maximum width or diameter thereof. The second end preferably has an internal constriction at its extremity, the first end being locatable within the internal volume of the second end and held by the constriction.

The first end is preferably radially compressible. In this embodiment, the first end may include at least one longitudinally extending slot allowing the first end to compress radially. The second end is advantageously shaped so as to cause a first end of an element coupled thereto to compress when fitted into or removed from the second end.

Preferably, the bore extending in the inter-cooperating elements has a smaller diameter at the first end relative to the second end. Advantageously, the control device has a diameter greater than the first end of the bore when radially compressed on insertion into or withdrawal form a second end of a coupled inter-cooperating element. With this structure, the inter-cooperating elements cannot be separated from one another until the control device is removed.

In another embodiment, the second end is radially expandable, for example by having one or more longitudinally extending slots therein.

The bore of each or at least one inter-cooperating element may have a uniform taper from the one end to the other end. In practice, this can be accomplished by the wall forming the bore being substantially straight in longitudinal cross-section. In some embodiments, the bore of each or at least inter-cooperating element has a non-uniform taper from the one end to the other end. In practice, this can be accomplished by the wall forming the bore being curved in longitudinal cross-section. The nature of the bore, or more particularly the shape of the wall or walls forming the bore is not critical to the functioning of the tapering characteristic of the bore.

It is preferred, though not essential, that the inter-cooperating elements have tapering bores in the same orientation along the length of the guide wire.

The inter-cooperating elements may have different lengths. For example, longer inter-cooperating elements may be located proximally along the guide wire and relatively shorter inter-cooperating elements located more distally along the guide wire. In this manner, the flexibility of the guide wire can differ along the length thereof, for example to be more flexible towards its distal end, by having shorter inter-cooperating elements, and less flexible towards its proximal end, by having longer inter-cooperating elements.

The control device may be a wire or cable connected at the distal end of the guide wire.

The control device may be made of a metal or metal alloy, of carbon fibre, of a strong polymer material or of fibrous material, either synthetic and natural. For larger diameter devices the control device may be made of a stretchable or elastomeric material such as silicone.

The assembly can provide a guide wire which can be very soft, that is able to flex sideways with very little resistance, yet able to be made more rigid or even locked in position. In its soft configuration, the guide wire is able to pass through tortuous vasculature without damaging or causing trauma to the vessel walls. The preferred embodiments are particularly suited to neurological applications, that is delicate cerebral vessels. In its generally rigid or locked configuration, the guide wire is able to provide a good support for stiffer elements of an introducer assembly. These may be any of the elements discussed above.

In the preferred embodiment, the control device applies a longitudinally extending constraining force on the inter-cooperating elements.

The control device is advantageously connected to a driving element at the proximal end of the guide wire. An example is a pull handle or the like. In other embodiments, the control device may be mechanically driven, such as by an electric motor, a spring and so on.

In some embodiments, the inter-cooperating elements snap-fit to one another.

There may be provided a covering sleeve or sheath disposed over the inter-cooperating elements. In practice, a sleeve can ensure a smooth outer surface to the guide wire.

Other aspects and advantages of the teachings herein will become apparent from the description of the preferred embodiments which follows.

### Brief Description of the Drawings

Embodiments of the present invention are described below, by way of example only, with reference to the accompanying drawings, in which:
Figure 1 is a schematic diagram showing the principle of operation of embodiments of the guide wire assembly disclosed herein;
Figure 2 is a schematic diagram of an example of guide wire assembly as taught herein;
Figure 3 is a side elevational view in partial cross-section of an inter-cooperating element of an embodiment of guide wire assembly;
Figure 4 is a side elevational view in partial cross-section of the inter-cooperating element of Figure 3 in a different orientational position;
Figure 5 is a perspective view of the inter-cooperating element of Figures 3 and 4;
Figure 6 is a side elevational view of two of the inter-cooperating elements of Figures 3 to 5 in the manner in which they could be coupled together to form the flexible portion of a guide wire;
Figures 7A to 7C are different views of an inter-cooperating element of another embodiment of guide wire;
Figures 8A to 8D are side elevational views of in cross-section of a series of inter-cooperating elements having similar the characteristics to the embodiment of Figures 7A to 7C;
Figure 9 is a perspective view of an embodiment of guide wire having a first example of sheath or cladding over the inter-cooperating elements; and
Figure 10 is a perspective view of an embodiment of guide wire having another example of sheath or cladding over the inter-cooperating elements.

### Description of the Preferred Embodiments

Various embodiments of medical guide wire assembly are described below and shown in the accompanying drawings. It is be understood that the drawings are schematic only and are not intended to show the various components of the assembly to scale. In many cases, the assembly has been depicted in enlarged form for the sake of clarity of disclosure. The skilled person will appreciate that the assembly may be configured to a variety of different sizes, and shapes, in order to correspond to the vessel or other organ in which the device is to be deployed. In practical embodiments, the guide wire assembly can be produced to have a very small diameter, including of less than a millimetre. Where dimensions are given in drawings these are solely for exemplary purposes. The elements may be formed to have different dimensions and different proportions.

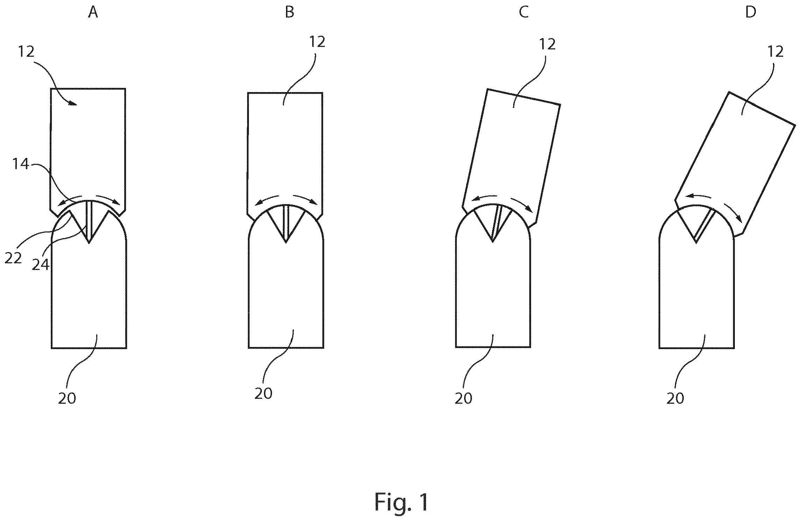

Referring first to Figure 1, this is a schematic diagram depicting the principle of operation, in general terms, of embodiments of guide wire taught herein.

The guide wire, which is in practice an assembly of components, includes a plurality of pivotable or rotatable elements of which two are shown in Figure 1. A first element 12 has a concave contact surface 14, preferably being part-spherical. The second element 20 has a convex contact surface 22, which is also part-spherical, and has a radius of curvature that matches or substantially matches the radius of curvature of the concave surface 14. The two contact surfaces 14, 22 face one another and may lie in planes which are perpendicular to the longitudinal axis of the device (when straight).

The surfaces 14, 22 in at least some embodiments are able to engage with one another in non-sliding manner so as to lock the elements 12 and 20 together non-rotatably. For this purpose, the surfaces 14 and 22 may be roughened, may have corresponding keying elements such as inter-engaging teeth or other relief patterns, may simply make friction contact with one another, and so on.

Each element 12, 20 has an internal channel or lumen therein, not visible in Figure 1, within which there is disposed a control device, which in this example is a wire or thread 24. The control device 24 is fixed at or proximate at least one end of the assembly of elements 12, 20 and is able to be loosened and tightened to change the flexibility of the guide wire and in the preferred embodiments to effect a selective locking action on the elements 12, 20. For this purpose, the most distal element 12, 20, at the distal tip of the guide wire, need not have a lumen passing therethrough and in the preferred embodiments simply fixes the distal end of the control device, for instance in a blind bore; although in other embodiments there may be provided a dedicated distal element for this purpose.

The left-most sketch of Figure 1, that is view A, shows the elements 12, 20 in a condition in which they are loosely arranged relative to one another, that is with the control device 24 in what could be described as a loose or unlocked configuration. In this state, the surfaces 14, 22 may be spaced from one another or otherwise in contact but with no or no appreciable contact force between them. The elements 12, 20 could be said to be in an open or relaxed configuration. In this configuration, the surfaces 14, 22 are not pressed together and are able to slide over one another, such that the elements 12, 20 can rotate or pivot relative to one another. When the contact surfaces 14, 22 are part-spherical, the elements 12, 20 are able to pivot or rotate in any direction relative to one another, in the manner of a universal joint.

The surfaces 14, 22 may, in some embodiments, have different shapes in order to limit their angular rotation relative to one another. For instance, the surfaces 14, 22 could be at least part-cylindrical so as to rotate around a single axis of rotation, or could be shaped to rotate along two or more axes of rotation, or the like. It is preferred, though, that the surfaces 14, 22 are part-spherical.

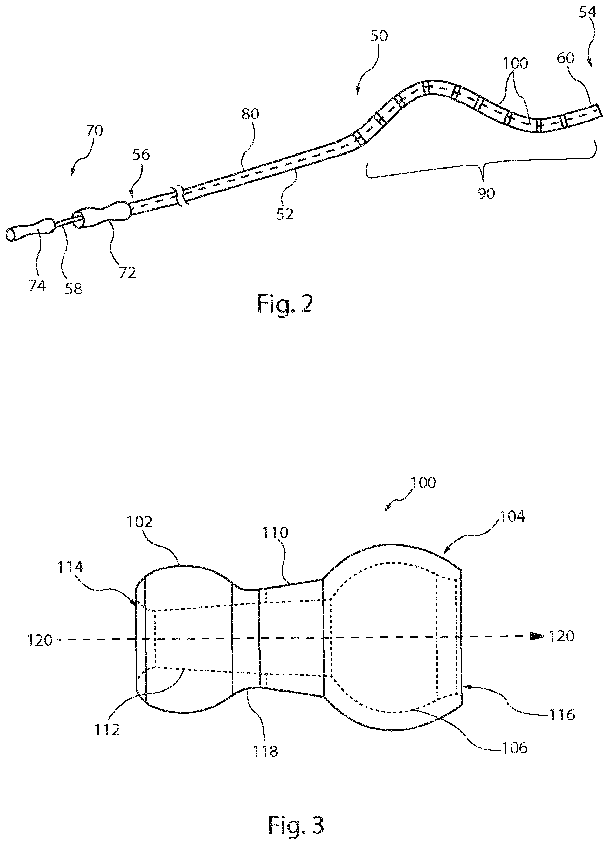

In sketch B of Figure 2, the control device 24 has been tightened in order to press the elements together and stiffen the guide wire. It may be tightened sufficiently so as to cause the surfaces 14, 22 together into locking engagement, that is such that the surfaces 14, 22 are not able to slide relative to one another, thereby fixing the guide wire in the configuration in which the elements 14, 22 form when locked to one another. The third and fourth sketches of Figure 1, that is sketches C and D, show the elements 12, 20 locked together at different angles. In practice, as will become apparent, the assembly can be rotated to any desired configuration within the permitted range of movement and then stiffened or locked in that configuration by tightening the control device 24. The assembly is such that the control device 24 can be released, loosened, again so to allow the elements 12, 20 to rotate freely once more relative to one another. In this manner, and as will become apparent below, the assembly can be selectively loosened to be flexible and stiffened or even locked in a chosen configuration. The skilled person will appreciate that the guide wire assembly taught herein need not be fixed in a totally immovable manner and that in some embodiments at least the elements may be movable even when the control device 24 is engaged, but only on the application of a higher force, for example notably larger that the forces imparted to it by the other elements of an associated introducer assembly which are fed over the guide wire.

Referring now to Figure 2, this shows in schematic form an example of a guide wire 50 having the characteristics taught herein.

The guide wire 50 includes an outer tubular element 52 having a distal end 54 and a proximal end 56. At the distal end 54 there may be provided a soft tip 60 made, for example, of a plastics or elastomeric material. In some embodiments the tip 60 may be a weld joint or other relatively rigid tip and in all cases is preferably rounded so as to be atraumatic. The proximal end 56 is attached to a handle assembly 70, of which an example of only the principal components is shown. Disposed within a lumen (not shown in Figure 2) of the guide wire assembly 50 is an elongate flexible control device 58 fixed at the distal end 54 of the guide wire and passing through to the handle assembly 70. The control device 58 is typically a wire or cable made, for example, of metal, carbon fibre, plastics material such as silicone or the like, and may be single stranded or multi stranded, as preferred.

In the embodiment shown in Figure 2 the control device 58 has a proximal end attached to a handle portion 74 of the handle assembly 70, which in its simplest form can be moved towards and away from a distal handle portion 72 fixed to the catheter 56. Moving the proximal handle portion 74 towards the distal handle portion 72 loosens the control device 58 and as a result loosens the connection between the segments or elements of the guide wire, enabling them to rotate relative to one another. On the other hand, moving the proximal handle portion 74 away from the distal handle portion 72 will tense the control device 58, which in practice will pull the segments of the guide wire assembly towards one another, increasing the rigidity or locking the guide wire. When the assembly is constructed to lock, tightening of the control device 58 will cause the facing surfaces of the segments of the guide wire to press against one another and lock to one another by way of friction or mechanical engagement. Once locked, the segments of the guide wire will not be able to rotate and the guide wire will therefore remain in that configuration.

There may be provided a spring or other biasing mechanism between the handle portions 72 and 74 to bias them apart from one another and therefore the assembly into a less flexible or locked condition. There may also, or in the alternative, be provided other control mechanisms such as locking pins, drive elements or the like for adjusting the tension of the control device and for fixing this during operation of the guide wire.

It is to be appreciated that Figure 2 shows a simple example of a locking mechanism and that in place of a spring other mechanisms could be used, such as a motor, a solenoid, a screw fitting, and so on. Any mechanism which is able to pull back the wire so as to tighten the pivoting/rotating elements of the guide wire can be used.

The main portion 80 of the guide wire 50 may be in the form of a catheter or cannula of unitary elongate form with a lumen extending therethrough, and with a distal end 100 formed of a series of inter-cooperating elements 100 of the types disclosed herein. The control device 58 extends through the lumen of the proximal portion 80 and through the internal, aligned, lumens of the elements 100 to the tip 54 of the guide wire 50. In this manner, the control device 58 can control the configuration of the guide wire, in the manner described in further detail below.

The proximal portion 80 of the guide wire 50 is preferably flexible and may be made in any conventional form and with known materials. Equally, in some embodiments, the portion 80 of the guide wire may be made from a rigid cannula.

In this example of Figure 2, only the distal end portion 90 of the guide wire is structured with rotatable elements 100 so as to have a varying flexibility, and preferably locking capability. In other embodiments, inter-cooperating elements 100 may be located elsewhere along the length of the guide wire, in dependence upon the desired nature of the guide wire, and may also extend through a major part of or the entire length of the guide wire 50. For example, in some cases the guide wire may be formed entirely of inter-cooperating elements, while in other embodiments an intermediate portion or the proximal portion may be so formed. These are design choices readily available to the skilled person formulate on the basis of the structure of the assembly and components taught herein.

In use, the guide wire 50 can be fed endoluminally through a patient's vasculature, with the control device 58 in its relaxed condition, that is with the segments 100 able to rotate relative to one another, such that the guide wire 50 is very flexible. In this condition, the guide wire 50 can pass through tortuous vasculature and also through delicate vessels including, for example, the neural vessels.

It will be appreciated that the elements or segments 100 will rotate relative to one another to allow the segmented portion 90 of the guide wire 50 to adopt with little resistance complex curved shapes, in dependence upon the direction of curvature and bending of the vessels through which it passes. The guide wire 50 can be stiffened or locked in the configuration in which it is variably curved, by pulling on, that is tightening, the control device 58. This can reduce the flexibility of the distal portion 90 and preferably lock the segments 100 relative to one another. Locking can be done for a variety of reasons. For instance, the guide wire elements 100 can be locked relative to one another to assist in feeding the guide wire 50 through a patient's vasculature, for example for directing the distal end 54 of the guide wire 50 through a bifurcation or into a branch vessel. After positioning, the guide wire sections 100 can be loosened again. The guide wire 50 can also be locked into its configuration once it has been positioned within a patient's vessels at the site at which treatment is to be carried out, so as to support an introducer assembly passed through the patient's vasculature over the guide wire 50.

Referring now to Figures 3 to 5, these show an embodiment of inter-cooperating element 100, for use in a guide wire assembly of the type shown in Figure 2 and taught herein. The embodiment of element 100 shown may be formed as a moulding or casting of any suitable material including metal, metal alloy, a plastics material and so on. The element 100 includes first and second ends 102 and 104 which are spaced apart by an intermediate zone or neck 110. Extending through the element 100 from the first to the second end is a lumen or bore 120. The element 100 is generally round in plan view.

The first end 102 has a rounded convex outer surface, which in the preferred embodiment is preferably part-spherical. At its extremity, the first end 102 has a generally flat surface 314 with, in the embodiment shown, an internally flared and rounded opening to lumen 120. The flared opening avoids any sharp edges which may damage or prematurely wear the control device 58.

The second end 304 is larger than the first end 102 and specifically has a rounded concave internal wall 106 having a shape which is preferably consistent with or substantially identical to the outer shape of the first end 102. The internal wall 106 defines a chamber having a diameter which is the same as or only slightly larger than the outer diameter of the first end 102, such that, as described below, the first end 102 of one element 100 can fit into the chamber of the second end 104 of another element 100.

The second end 104 has an outer wall, which in this example is rounded in similar manner to the internal wall 106, that is preferably part-spherical. The outer shape of the second end 104 does not affect the functioning of the element 100 but that shown reduces the amount of material needed for the element, thereby reducing volume and weight, and provides a smooth rounded outer surface to the assembly.

The second end 104 terminates in a flat end 116 and, as depicted in the drawings, the inner wall 106 of the second end 104 curves smoothly at the opening 316 towards the outer surface of the second end 104.

The neck 110, in this embodiment, has an outer surface which tapers from the second end 104 to the first end 102 and terminates in a rounded collar 118 leading to the first end 102.

The bore formed by the internal wall 112 widens from the opening 114 of the first end 102 to the junction of the internal wall 306 of the second end 104 and in practice has a frusto-conical shape.

The outer surface of the neck 110 widens in a similar manner to the internal bore 110, although this is not a necessary characteristic. In fact, in other embodiments, the outer surface of the neck 110 need not taper and may, for example, be cylindrical.

The combination of the internal wall 112 and the internal surface 106 of the second end 104, together with the open ends, provides an open lumen which extends through the entire length of the element 100.

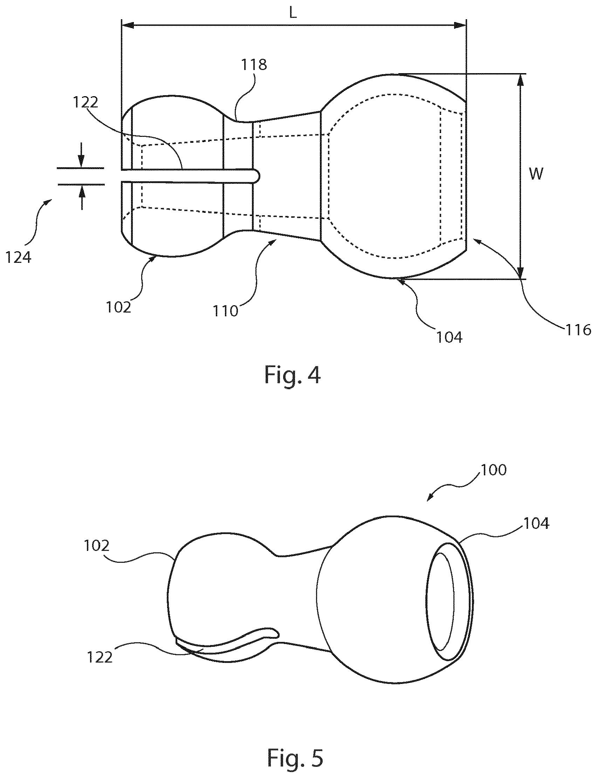

Referring now specifically to Figure 4, this shows the element of Figure 3 rotated by 90 degrees around the axial centreline of the bore 120. As can be seen, in this embodiment the first end 102 is provided with two longitudinally extending slots 122 which extend to the neck 118. Other embodiments may have more than two slots 122, preferably evenly spaced radially around the first end 102.

The maximum diameter of the outer surface of the first end 102 is preferably larger than the minimum diameter of the opening 116 of the second end 104, by preferably no more than the width 124 of the slots. The first end 102 is resiliently deformable, that is compressible, such that it can be pushed into the opening 116 of the second end 104 of a mating element 100. The skilled person will understand that the walls of the first end 102 can also change shape, by radial compression, as a result of the provision of the slots 122. In other embodiments the element 100 may be made of a naturally resilient material and in some circumstances resilient enough that slots are not necessary.

In other embodiments, the second end 104 may additionally or alternatively be provided with longitudinally extending slots similar to the slots 122, although the arrangement shown in Figures 4 and 5 is preferred.

Figure 5 shows a perspective view of the element 100, in which the rounded, part-spherical shape of the ends 102 and 104 can be clearly seen.

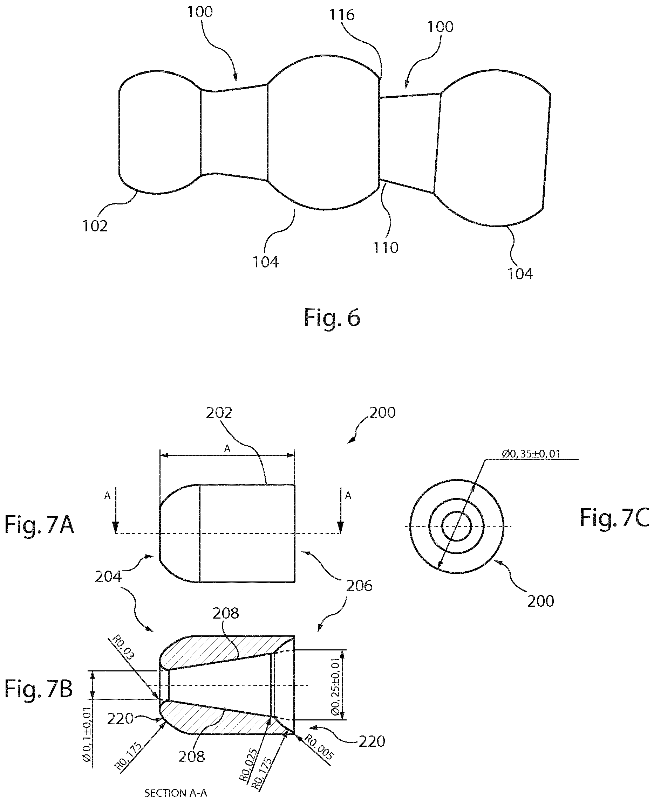

Two or more elements 100 can be fitted together in series, in the manner shown in Figure 6. As can be seen in this Figure, the first end 102 of one element fits into the second end 104 of an adjacent element 100 and in such a manner that the opening 116 of the second end is spaced from, that is wider than, the neck 110 of the element coupled thereto. This gap enables the two elements 100 to pivot relative to one another, giving the assembly the ability to curve or bend. The rounded nature of the elements 100 enables them to pivot in any radial direction, giving the assembly an infinite number of directions of bending.

Any number of elements 100 can be coupled together in dependence upon the desired characteristics for the guide wire 50. In this regard too, while Figures 3 to 6 show a single size of element 100, with a length L and maximum width W, it is envisaged that the elements could have a varying length (some longer, others shorter) which in practice will alter the bending characteristics of the guide wire 50. Similarly, the elements could be constructed to have a varying maximum diameter W if desired, for instance so as to allow the guide wire assembly to be narrower at its distal end.

The lumen 120 accommodates a control device 58, typically a wire, of the type described elsewhere herein. The control device 58 will extend through the inter-cooperating elements 100 to the distal extremity 54 of the guide wire and typically be attached to a distal most element 100 or to a tip 60 of the assembly. As explained above, the proximal end of the control device 58 is attached to a handle assembly 70.

In the preferred embodiments, the control device 58 has a diameter which is the same as or only slightly smaller than the diameter of the bore 120 at its narrow end, that is at the opening 114 of the first end 102. Any diameter difference is preferably less than the amount by which the first end 102 must compress to slide into or out of the second end 104. In this manner, when the control device 58 is fitted into the lumen, the elements 100 cannot separate form one another because there is no space for the first ends 102 to compress to be able to be pulled out of the opening 116 of the second ends 104. This provides a mechanism for securely attaching the elements 100 together and preventing their separation, for example when the guide wire 50 is being pulled out of a patient.

The elements 100 shown in Figures 3 to 6 enable pivoting in 360° and as a result provide a guide wire which can curve and bend in any direction within a vessel or network of vessels. The flexible section 90 formed by the elements 100 can therefore be curved in a variety of different directions and a multitude of times over its length.

The surfaces are the inter-cooperating elements 100, in particular the outer surface of the first end 102 and the inner surface 106 of the second end 104, may be smooth, textured, toothed or friction surfaces, for the purposes herein described. The outer diameter of the first end 102 is preferably less than the inner diameter of the chamber 106 of the second section 104 such that when the first end 102 of section 100 is fitted into the second end 104 of an adjacent inter-cooperating element 100, the two elements 100 are able to pivotal, or rotate, relative to one another easily. In this case, the control device 58 is held in a relatively loose configuration.

When the control device 58 is tightened, by suitable operation of the handle assembly 70, in the example shown by pulling the end element 74 in a proximal direction that is away from the handle element 72, the elements 100 will be pressed together. In this manner, the outer surface of the first end 102 would be pressed against the inner surface 106 of the second end 104 in which it is located. Where the surfaces are smooth, the pressure imparted on the surfaces will reduce their ability to slide relative to one another and will therefore stiffen the guide wire section 90. Where the surfaces are roughened, textured or otherwise friction surfaces, as they are pressed together the guide wire will lock, preventing any further pivoting or rotation of the elements 100 relative to one another and as a result will lock the guide wire section 90. As a result, the section 90 of the guide wire 50 will retain the shape it has when it is locked, providing a solid and stable support for any further devices fed over the guide wire.

When the control device 58 is loosened, particularly by bringing the proximal handle portion 74 closer to the distal handle portion 72, the guide wire section 90 will loosen as the elements 100 no longer press against one another and the relevant surfaces are able to slide relative to one another again. The guidewire section 90 will therefore retain its maximum flexibility.

It is not necessary for the elements 100 to lock tight to one another when the control device 58 is fully deployed, although it is preferred if this occurs, in which case the control device 58 could be considered to be a locking device.

Referring now to Figures 7A to 7C, these show another embodiment of inter-cooperating element 200 according to the teachings herein. The element 200 may be made with the same materials as the element 100 of the embodiments of Figures 3 to 6, but has a different shape, namely a body member 202 which is substantially cylindrical, a first end 204 with a rounded convex surface and a second end 206 with a rounded concave surface. An internal wall 208 of frusto-conical shape extends from the first end 204 to the second end 206 and widens from the first to the second end. The frusto-conical internal wall 208 is open at the two extremities of the element 200, such as to have first and second openings 210, 220 and as a result a lumen passing through the length of the element 200. At the first end 204, the internal wall 208 is rounded, as shown for example in Figure 7.

Preferably, the internal wall at the second end 220 has a curvature which is the same as or approximates the curvature of the outer surface of the first end 204, such that the first end 204 of one element 200 can fit into the concave recess formed by the internal wall at the second end 206 of an adjacent element 200. When so positioned, the elements 200 provide an internal lumen for receiving a control device 58 for the type disclosed herein.

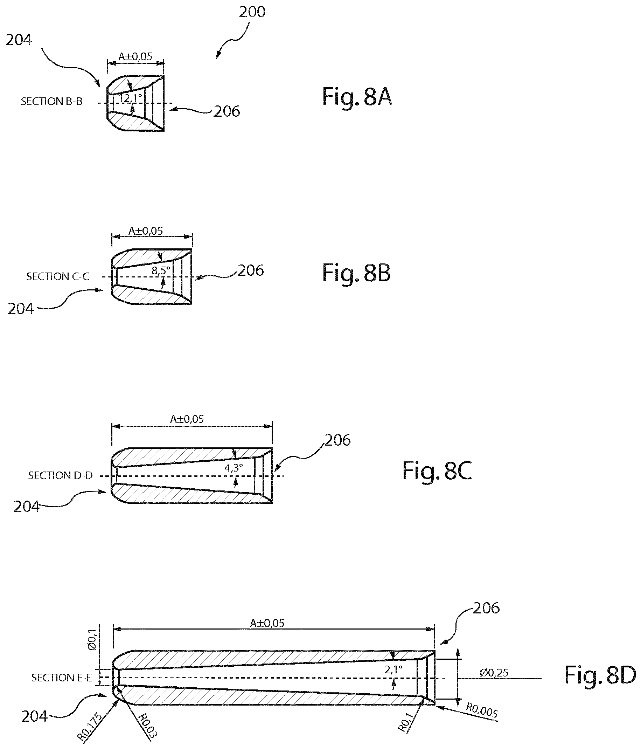

With reference to Figure 8, this shows a series of inter-cooperating elements 200 having the same fundamental characteristics as the example on Figure 7, save for the fact that the elements of Figures 8A to 8D all have different lengths. They all have, however, the same diameter and in the preferred embodiment first and second ends 204, 206 that are equivalent to the ends 204 and 206 shown in Figure 7.

The bore formed by the internal wall 206 of the different versions of element 200 shown in Figures 8A to 8D has a varying taper angle to accommodate their different lengths. For instance, in the version of Figure 8A, the internal wall has an angle of taper of 12.1°, while that of Figure 8B has an internal taper angle of 8.5°. The example of Figure 8C has an internal taper angle of 4.3°, while that of Figure 8D has an internal taper angle of 2.1°.

With the examples of elements 200 shown in Figures 8A to 8D, it is possible to construct a guide wire 50 with elements of a variety of lengths, which will cause the guide wire 50 to have different flexure characteristics along its length. Particularly, in a portion of the guidewire 50 formed of longer elements such as those shown in Figure 8D, the guide wire will be relatively inflexible, whereas in portions made up of elements 200 of shorter length, the guide wire will be able to bend more. In a typical embodiment, a guide wire assembly may include longer inter-cooperating elements 200 in its proximal portion and progressively shorter elements 200 towards its distal end. In this manner, the guide wire 50 can be made more flexible towards its distal end, useful in navigating through tortuous vessel systems.

As with the embodiments described above, the surfaces of the first and second ends 204, 206 of the elements 200 may be smooth or non-smooth, such as roughened, textured or otherwise high friction surfaces. In this manner, a guide wire made up of sections 200 may exhibit the operational characteristics described above in connection with the embodiment of Figures 3 to 6. The guide wire can, in these circumstances, be configured between a relatively flexible configuration to a relatively stiff configuration and also may be locked in position in at least some embodiments.

The internal taper of the bore or lumen shown in the various embodiments of inter-cooperating elements 100, 200 disclosed herein provides room for the control device 58, particularly when the guide wire elements 100, 200 are pivoted so as to cause the guide wire to bend or curve. In the absence of a tapering internal lumen, either the assembly must be provided with a relatively large lumen, which restricts the dimensions to the guidewire and weakening the assembly, or will impart a straightening effect on the inter-cooperating elements 100, 200. The tapering internal lumen, therefore, aids in maintaining the guide wire in a bent configuration even as the control device 58 is tightened to retain a curved shape of the guide wire.

In Figures 7 and 8 various dimensions for the elements 100, 200. It is to be understood that these dimensions are exemplary only and that the eventual dimensions of the elements will be dependent upon the desired dimensional characteristics of the guide wire.

The bore of each or at least one inter-cooperating element 100, 200 may have a uniform taper from the one end to the other end. In practice, this can be accomplished by the internal wall of the element that forms the bore being substantially straight in longitudinal cross-section, as can be seen for example with the embodiments of Figures 8A to 8D. In some embodiments, the bore of each or at least inter-cooperating element 100, 200 may have a non-uniform taper from the one end to the other end. In practice, this can be accomplished by the wall forming the bore being curved in longitudinal cross-section. The nature of the bore, or more particularly the shape of the wall or walls forming the bore is not critical to the functioning of the tapering characteristic of the bore.

As explained above, in the preferred embodiments the assembly is provided with a covering sheath or cladding over at least that part of the guide wire assembly formed of the inter-cooperating elements 100, 200. This can ensure that the guide wire maintains a smooth outer surface even when it is curved, and also ensures that the elements 100, 200 remain coupled to one another, for instance on breakage of the internal control wire. The sheath or cladding may also provide lubricity to the guide wire, for instance when made of or covered by a lubricious material.

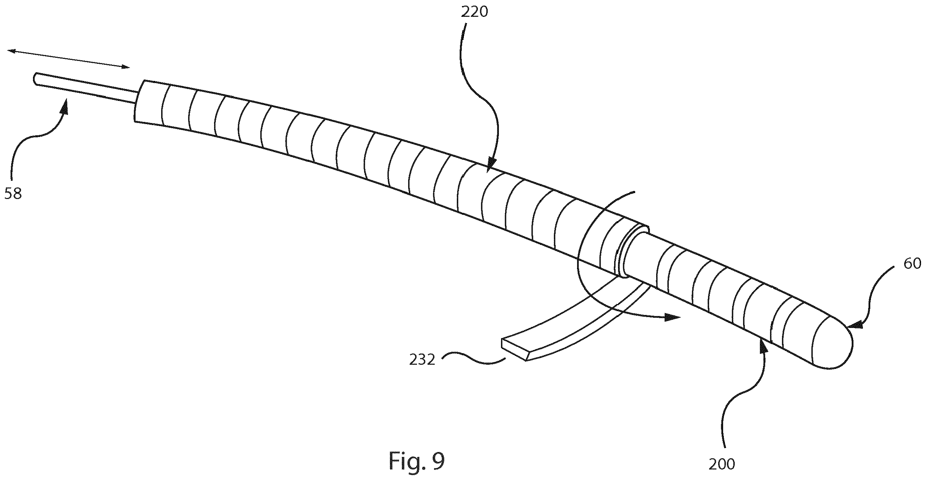

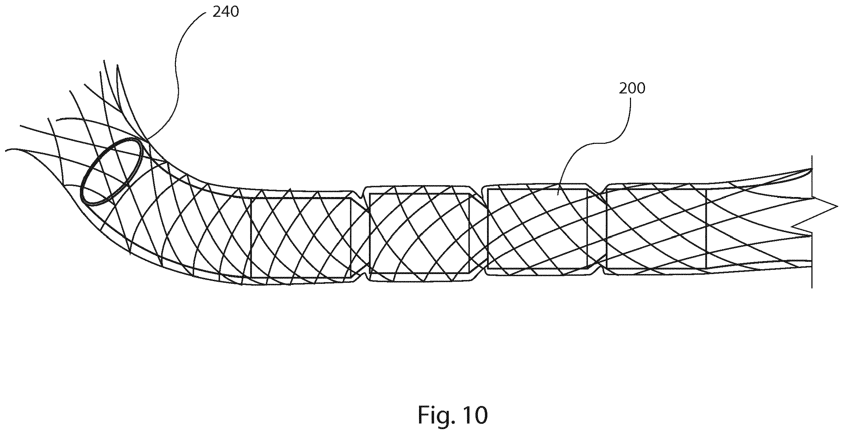

Referring first to the embodiment of Figure 9, this shows a guidewire formed of a series of inter-cooperating elements 200 of the type shown in Figures 7 and 8, fitted over a control wire 58 coupled at its distal end to a rounded distal tip 60, as previously described. It will be appreciated that Figure 9 (and equally Figure 10) shows only one of many embodiments and that the guidewire could be formed of the elements of the type shown in Figures 3 to 6 or of any of the other forms of elements described and contemplated in this specification.

The assembly shown in Figure 9 includes a cladding 220 that is wrapped around at least the section of the guide wire formed of the elements 220. The cladding 220 may be provided over a greater extent of the guide wire and in some embodiments around the entire length of the guidewire, that is including sections not formed of the inter co-operating elements. The cladding 220 is, in this embodiment, a strip of pliant material wrapped helically around the core of the guidewire in the manner depicted at 232. The arrow depicts the direction of unwinding of the cladding strip, simply to illustrate how this is fitted to the guidewire.

The cladding 220 preferably extends all the way to the tip 60, leaving the tip 60 exposed. In practice, as will be appreciated, the strip 232 will be less than 1 mm in thickness, preferably of no more than 0.05 to 0.50 mm, so as not to contribute unduly to the diameter of the assembly. The strip 232 may be made of any suitable material of which many are available in the art. Examples include a polymer material such as a polyamide (for instance Nylon), polyethylene terephthalate (PET), a polyether block amide (such as PEBAX); or a metal material such as nitinol, platinum, stainless steel or a combination of polymer and metal. The cladding 220 could advantageously be made of a lubricious material or be provided with a lubricious coating, such as a Hydrofil coating.

The strip 232 may be laid edge to edge along the length of the guidewire or may be laid with the edges overlapping.

The strip 232 may be simply wound tightly over the inter co-operating elements 200, although some embodiments may be attached to itself or to the core of the guide wire, for example by a suitable adhesive.

As explained, the cladding 220 provides a smooth outer surface to the guidewire and also ensures continued integrity of the assembly during its use and in the unlikely event that the control wire 58 were to break.

Figure 10 shows another embodiment of guidewire in which the inter-cooperating elements 200 are housed within a sleeve 240 made of braided wire. The braided sleeve 240 extends along the entire length of the section formed by inter-cooperating elements 200 and in some embodiments also over other parts of the guidewire assembly, including for instance all the way to the proximal end of the guidewire. As with the previously described embodiments, the braided sleeve preferably extends to and terminates at the rounded tip 60 (not shown in Figure 10). The braided sleeve 240 may be made of one or more wires.

It is not necessary for the braided sleeve 240 to be attached to the inter-cooperating elements 200, allowing the elements 200 to slide within the sleeve 240 and providing optimum flexibility of the assembly.

The braiding may be formed of any suitable metal or metal alloy, stainless steel, platinum and Nitinol being preferred.

The assembly can be provided with radiopaque properties. This may be in the form of an additive or filler to the cladding and/or to the material used in the formation of the inter co-operating elements 100, 200.

The guidewire assembly may be provided with a proximal portion not formed of inter co-operating elements of the types disclosed herein. In this case, the proximal portion is preferably made of a flexible tubular element of the type typically used for catheters. It may also be made as a hypotube, for instance of stainless steel, Nitinol or other suitable material. The hypotube may be transversely slotted, in known manner, to increase its flexibility.

With the arrangement disclosed it is possible to provide a guide wire with a soft locking section and having selectively varying flexure characteristics.

The control device may be a metal or metal alloy wire or a wire made from, for example, an ultrahigh molecular weight polyamide such as Dynema (TM).

The elements 100, 200 may be made of metal or a metal alloy but in other embodiments may be made of a polymer material, such as a polyamide (for example Nylon), or any other suitable and preferably biocompatible material.

In practice, the assemblies taught herein can provide a very soft guide wire able to be made more rigid or locked into a shape during a medical procedure. The guide wire can retain that shape and in effect become stiff when locked. This can provide more optimal support for a microcatheter introduced over the guide wire. The guide wire can be manipulated, unlocked or otherwise made soft again and this can be controlled entirely by the clinician and repeated over and again during the procedure. In other words, the guide wire can be softened and stiffened or locked repeatedly as required.

The guide wire may have an outer diameter in the region of 0.35 mm to around 0.9 mm for neurovascular procedures but could equally have greater diameters, for instance of a millimetre or more, for other vessels. The portion of the guide wire formed of inter-cooperating elements may have a length of a few centimetres up to around 5 to 10 centimetres in some instances. The skilled person will appreciate that in many cases it is a design choice how long to make the lockable part of the guide wire.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in British patent application number 1720416.5, from which this application claims priority, and in the abstract accompanying this application are incorporated herein by reference.

## Claims

1. A medical guide wire of elongate form and having a longitudinal dimension, a distal end and a proximal end; at least a portion of the guide wire being formed of a plurality of inter-cooperating elements disposed longitudinally in series, the inter-cooperating elements having cooperating facing surfaces able to slide relative to one another so as to cause the guide wire to be bendable in at least one lateral dimension; each inter-cooperating element having a bore extending from one end to the other end thereof, the bore having a taper from said one end to the other end; and a control device extending through the bores of the inter-cooperating elements; the control device having at least one operating state enabling the inter-cooperating elements to slide relative to one another.

2. A medical guide wire according to claim 1, wherein the control device has a first operating state in which the inter-cooperating elements can slide relative to one another and the guide wire is relatively flexible and a second operating state limiting sliding of the inter-cooperating elements and the guide wire is relatively less flexible.

3. A medical guide wire according to claim 2, wherein the control device has an operating state preventing sliding of the inter-cooperating elements and the guide wire is stiff.

4. A medical guide wire according to any preceding claim, wherein the cooperating facing surfaces are textured, toothed or friction surfaces.

5. A medical guide wire according to any preceding claim, wherein one or both of the cooperating facing surfaces is curved, rounded or part-circular.

6. A medical guide wire according to any preceding claim, wherein cooperating facing surfaces are respectively convex and concave.

7. A medical guide wire according to any preceding claim, wherein each inter-cooperating element includes at one end a convex surface and at the other end a concave surface.

8. A medical guide wire according to any preceding claim, wherein the inter-cooperating elements include a first end with a rounded outer surface and a second end with an inner surface, the second end being sized to envelop the first end of an adjacent inter-cooperating element.

9. A medical guide wire according to claim 8, wherein the first end is radially compressible.

10. A medical guide wire according to claim 9, wherein the first end includes at least one longitudinally extending slot allowing the first end to compress radially.

11. A medical guide wire according to claim 10, wherein the bore extending in the inter-cooperating elements has a smaller diameter at the first end relative to the second end.

12. A medical guide wire according to claim 11, wherein the control device has a diameter greater than the first end of the bore when radially compressed on insertion into or withdrawal from a second end of a coupled inter-cooperating element.

13. A medical guide wire according to any preceding claim, wherein the bore of each or at least one inter-cooperating element has a uniform or non-uniform taper from the one end to the other end.

14. A medical guide wire according to any preceding claim, wherein the inter-cooperating elements have tapering bores in the same orientation along the length of the guide wire.

15. A medical guide wire according to any preceding claim, including inter-cooperating elements of different lengths.

16. A medical guide wire according to claim 15, wherein longer inter-cooperating elements are located proximally relative to shorter inter-cooperating elements along at least part of the length of the guide wire.

17. A medical guide wire according to any preceding claim, including a covering or sleeve disposed over the inter-cooperating elements.
